# FASCICULE DE BREVET EUROPEEN

(11) **EP 4 152 960 B1**
(45) Date de publication et mention de la délivrance du brevet: **06.05.2026**
(21) Numéro de dépôt: 21732969.7
(22) Date de dépôt: 18.05.2021
(51) Int. Cl.: A61K 8/362, A61K 8/9789, A61Q 19/08, A23F 5/14, A23F 3/16, A61K 31/353, A61K 36/82, A61K 8/49

(54) **COMPOSITION D'ENRICHISSEMENT DE PRODUITS ALIMENTAIRES, COSMÉTIQUES ET/OU DERMATOLOGIQUES**
ZUSAMMENSETZUNG ZUR ANREICHERUNG KOSMETISCHER, DERMATOLOGISCHER UND/ODER LEBENSMITTELPRODUKTE
COMPOSITION FOR ENRICHING COSMETIC, DERMATOLOGICAL AND/OR FOOD PRODUCTS

(30) Priorité: 18.05.2020 FR 2004954
(43) Date de publication de la demande: 29.03.2023
(73) Titulaire: Black Idol, 80120 Vron (FR)
(72) Inventeur: LION, Benoît, 78220 VIROFLAY (FR); DE FRANCE, Jérôme, 80120 NAMPONT (FR)
(74) Mandataire: Yes My Patent
(86) Numéro de dépôt international: PCT/FR2021/050875
(87) Numéro de publication internationale: WO 2021/234281

(56) Documents cités:
- EP-A1- 2 008 524
- EP-A1- 3 488 849
- WO-A2-2007/113008
- CN-A- 109 589 323
- JP-A- 2009 136 223
- JP-A- 2011 019 463
- KR-A- 20170 017 025
- DATABASE GNPD [online] MINTEL; 13 April 2018 (2018-04-13), ANONYMOUS: "14 Day Detox Coffee Capsules", XP055768271, retrieved from https://www.gnpd.com/sinatra/recordpage/5563571/ Database accession no. 5563571
- DATABASE GNPD [online] MINTEL; 13 October 2014 (2014-10-13), ANONYMOUS: "Americano Sweet Portion Coffee", XP055768273, retrieved from https://www.gnpd.com/sinatra/recordpage/2722047/ Database accession no. 2722047
- DATABASE GNPD [online] MINTEL; 19 October 2010 (2010-10-19), ANONYMOUS: "Ground Coffee", XP055768478, retrieved from https://www.gnpd.com/sinatra/recordpage/1423830/ Database accession no. 1423830
- DATABASE GNPD [online] MINTEL; 21 May 2012 (2012-05-21), ANONYMOUS: "Americano Black Coffee", XP055768277, retrieved from https://www.gnpd.com/sinatra/recordpage/1802463/ Database accession no. 1802463
- HENNING SUSANNE M ET AL: "Bioavailability and antioxidant activity of tea flavanols after consumption of green tea, black tea, or a green tea extract supplement", AMERICAN JOURNAL OF CLINICAL NUTRITION, vol. 80, no. 6, 1 December 2004 (2004-12-01), pages 1558 - 1564, XP093234414, ISSN: 0002-9165, Retrieved from the Internet <URL:https://pdf.sciencedirectassets.com/782868/1-s2.0-S0002916504X80060/1-s2.0-S0002916522037406/main.pdf?hash=33a9f9acd84b8d6b8833201069a9779e2ca703ea4d3cae9ca3e94cbedec5b203&host=68042c943591013ac2b2430a89b270f6af2c76d8dfd086a07176afe7c76c2c61&pii=S0002916522037406&tid=spdf-e5c128b1-3db9-4908-b97a-c04> DOI: 10.1093/ajcn/80.6.1558
- DATABASE GNPD [online] MINTEL; 13 April 2018 (2018-04-13), ANONYMOUS: "14 Day Detox Coffee Capsules", XP055768271, Database accession no. 5563571
- DATABASE GNPD [online] MINTEL; 19 October 2010 (2010-10-19), ANONYMOUS: "Ground Coffee", XP055768478, Database accession no. 1423830
- DATABASE WPI Week 201954, Derwent World Patents Index; AN 2019-350914
- DATABASE WPI Week 201722, Derwent World Patents Index; AN 2017-13927H
- DATABASE WPI Week 201111, Derwent World Patents Index; AN 2011-B28444

## Description

La présente invention concerne une composition liquide d'enrichissement de produits alimentaires, de compléments alimentaires, de produits cosmétiques, et/ou dermatologiques. L'invention concerne en particulier une composition liquide aqueuse ou hydro-alcoolique pour l'enrichissement en extrait thé vert. L'invention concerne également l'utilisation de ladite composition liquide et le procédé de fabrication de ladite composition liquide. L'invention est définie par les revendications annexées.

### ART ANTERIEUR

Le thé vert, en particulier les feuilles du théier vert *Camellia sinensis* en infusion, est la boisson la plus consommée au monde après l'eau, et offre des bienfaits importants pour la santé. Les composés que l'on sait être responsables de ces bienfaits sont les polyphénols et en particulier les catéchines.

Les principaux polyphénols du thé vert (flavan-3-ol) sont les catéchines, c'est-à-dire la (+)-catéchine (C) et son stéréo-isomère et quatre dérivés, à savoir la (-)-épicatéchine (EC), la (-)-épigallocatéchine (EGC), le (-)-épigallocatéchine-3-gallate (EGCG), le (-)-épicatéchine-3-gallate (ECg).

Bien que les quantités de catéchines varient en fonction des facteurs qui influencent le métabolisme des plantes, tels que la lumière, la température, la disponibilité en nutriments, l'âge des feuilles et la composition génétique, elles constituent généralement 20 à 30 % de la masse de matière sèche des feuilles de thé vert fraîches sur la masse totale. Les catéchines représentent environ 80 % de la teneur totale en polyphénols du thé vert. Comme elles sont rarement détruites lors de la fabrication du thé vert, les catéchines constituent une part importante des extraits commerciaux de thé vert. En outre les catéchines sont moins oxydées dans le thé vert que dans les autres thés (gris et noirs).

Les catéchines du thé vert présentent plusieurs avantages pour la santé qui sont souvent associés à leurs activités antioxydantes, notamment le piégeage des espèces réactives d'oxygène et d'azote, la chélation des métaux lourds, et l'inhibition des enzymes oxydatives telles que la lipoxygénase et la cycloxygénase.

L'EGCG est le composé le plus intéressant parmi les catéchines mentionnées ci-dessus car il présente un fort effet antioxydant. En outre, il a été démontré que l'EGCG a un effet antimutagène, un effet antibactérien et également un effet bénéfique sur le taux de cholestérol dans le sang. Les autres catéchines présentes dans le thé sont beaucoup moins efficaces.

Les effets des polyphénols, et en particulier de l'EGCG, sur la santé dépendent de la quantité consommée mais aussi et surtout de leur biodisponibilité. Cependant, leur biodisponibilité est faible, ce qui semble être liée à une instabilité particulière de ces composés dans des conditions physiologiques. En particulier l'EGCG est peu soluble et difficile à dissoudre dans l'eau ce qui rend difficile son utilisation pour la supplémentation de produits alimentaires tels que des boissons ou des produits cosmétiques tels que des crèmes.

Une stratégie possible pour augmenter la stabilité des catéchines du thé vert et en particulier de l'EGCG est de les protéger par des antioxydants, ce qui permet d'éviter les changements chimiques causés par l'exposition à l'oxygène. Le document de brevet US2006057230 a proposé une méthode pour augmenter la biodisponibilité des polyphénols du thé, en administrant un polyphénol du thé par voie orale après un jeûne d'une nuit avant la prise de polyphénol du thé. Cependant, il n'y a aucune solution permettant d'améliorer la biodisponibilité des polyphénols du thé, et en particulier l'EGCG sans période de jeûne.

En effet, G. Williamson et al, Int.J.Vitam. Nutri Res. 77 (3) 2007, 224-235 ont démontré que l'ajout d'antioxydants (vitamines et caroténoïdes) n'avait aucun effet sur la biodisponibilité des polyphénols tels que les catéchines.

Le document EP 2008 524 divulgue un procédé de production d'un extrait de thé vert purifié contenant des catéchines non-polymères afin d'améliorer le goût de l'extrait de thé vert purifié en réduisant l'amertume et l'astringence dudit thé vert.

L'extrait de thé y est traité par l'enzyme tannase permettant, comme connu de l'art antérieur à la date de ce document, d'hydrolyser des esters de catéchine, tout en précipitant, afin éliminer les résidus astringents/amers, fibreux et résidus protéiques, par un mélange d'alcool et d'eau et présentant une forte proportion en alcool. L'extrait obtenu est sous forme de poudre.

Cependant, ce document ne décrit pas une composition d'extrait de thé vert enrichie en EGCG, et l'enrichissement en catéchines de café moulu, ne peut se faire directement par l'ajout d'une telle poudre sèche d'extrait de thé vert, car la différence de distribution granulométrique entre les grains de café moulu et la poudre fine et pulvérulente dudit extrait de thé vert amènerait à des problèmes de dispersion et de répartition de ladite poudre dans les grains de café moulu. La préparation nécessiterait alors impérativement une étape d'homogénéisation des deux phases de poudres.

Ainsi il est souhaitable que cet enrichissement en extrait de thé vert, en particulier en catéchines, et de manière particulièrement préférée en EGCG puisse être réalisé par une composition sous forme liquide pour en faciliter l'addition et la dispersion dans le produit alimentaire, compléments alimentaires, cosmétiques ou dermatologiques, ce qui complique le problème technique, l'EGCG étant peu soluble dans l'eau.

Enfin dans le cas particulier de l'enrichissement de café moulu, le problème technique est encore complexifié du fait que l'humidité apportée lors du procédé d'enrichissement du café par l'extrait liquide de thé vert peut sensiblement favoriser la croissance des champignons (moisissure), en particulier en raison de la mise en capsule de ce café formant ainsi une enceinte close empêchant l'élimination d'eau, développant alors des arômes désagréables à la dégustation et potentiellement nocif pour la santé.

Des capsules de café moulu comprenant des extraits de thé vert sont retrouvés dans le commerce, par exemple « Fit Coffee ». Ces capsules de café arguant des propriétés détoxifiantes contiennent un mélange d'ingrédients naturels, et notamment de l'extrait de thé vert contenant naturellement des catéchines.

Cette capsule ne revendique cependant pas des propriétés liées aux catéchines et à leurs teneurs, qui ne sont pas abordées dans la fiche produit, et ne paraît pas contenir l'(-)-épigallocatéchine-3-gallate (EGCG). Ainsi, les propriétés antioxydantes de ce produit restent discutables et ne paraissent pas être liées aux catéchines plus qu'aux autres produits contenus dans la formulation, et il perdure ainsi un besoin d'apport supérieur d'antioxydant au consommateur.

Le produit « Day Detox Coffee capsules » (GNPD Mintel base de données, ID d'enregistrement: 5563571, publié en avril 2018) décrit un procédé d'enrichissement de café en poudre dans une capsule de café avec du thé vert avant la fermeture de la capsule.

Le produit « Ground Coffee » (GNPD Mintel base de données, ID d'enregistrement: 1423830, publié en octobre 2010) décrit un mélange de café 100 % arabica infusé avec des antioxydants naturels retrouvés dans le thé vert.

Le document EP3488849 divulgue une solution aqueuse pharmaceutique stable comprenant de l'EGCG. Cette solution peut notamment être utilisée dans le traitement de cancers, d'un désordre cardiovasculaire, de diabètes, de maladies neurodégénératives ou de maladies dermatologiques.

Le document WO2007/113008 divulgue une capsule de café comprenant de la poudre de café enrichie avec de l'extrait de thé vert comprenant de l'EGCG.

La présente invention a donc pour objet de résoudre les problèmes susmentionnés en fournissant une composition permettant l'enrichissement facilité de produit alimentaire, compléments alimentaires, mais aussi cosmétiques ou dermatologiques, en particulier du café moulu encapsulé, en extrait de thé vert, en particulier en catéchines, et de manière particulièrement préférée en EGCG avec une stabilité et une biodisponibilité améliorée, et à des quantités efficaces pour obtenir l'effet souhaité, en particulier sur la santé.

Il a maintenant été trouvé par la Demanderesse qu'un mélange spécifique d'eau, éventuellement additionné d'éthanol, avec un extrait de thé vert comprenant des catéchines, en particulier de l'EGCG, et d'acide succinique permettait l'obtention d'une composition liquide résolvant ces problèmes techniques. En particulier, la Demanderesse a démontré que le mélange d'eau, éventuellement additionné d'éthanol, avec un extrait de thé vert forme un mélange pâteux-opaque qui se transforme en un liquide transparent et nettement plus fluide une fois additionné d'acide succinique.

Cet effet inattendu a, dans une large mesure, contribué à rendre plus précis et reproductibles les volumes de liquide ajoutés aux produit alimentaire, compléments alimentaires, cosmétiques ou dermatologiques.

Un autre effet inattendu lié à cette composition liquide est la très rapide évaporation après contact avec le café moulu. Un agrégat sec est constaté après ouverture des doses de café ainsi fabriquées, sans trace de moisissures dans le temps.

La présente invention a donc pour objet de résoudre ces problèmes techniques en offrant une composition liquide composée d'un extrait de thé vert riches en antioxydants contenant des catéchines de type EGCG et permettant d'enrichir lesdites capsules de café moulu, afin de répondre audits problèmes précités, d'une part, en rendant l'extrait de thé vert beaucoup plus fluide grâce à l'utilisation d'acide succinique, afin de pénétrer plus rapidement dans la capsule de café moulu, et d'autre part, en séchant rapidement les solvants utilisés de sorte qu'ils ne puissent pas catalyser la formation de moisissure avant la fermeture desdites capsules.

Ainsi un premier objet de la présente invention conceme une composition liquide comprenant ou consistant en :
- entre 15 et 60 %, de préférence entre 20 et 45%, en poids total de la composition d'extrait de thé vert, ledit extrait comprenant de préférence entre 10 et 50%, de préférence entre 10 et 40%, de manière plus préférée entre 15 et 35%, en poids total de la composition de catéchines, comprenant de préférence entre 5 et 40%, de préférence entre 5 et 25%, de manière plus préférée entre 9 et 20 % en poids total de la composition d'EGCG;
- entre 0,5 et 5,5%, de préférence entre 1 et 3 %, en poids total de la composition d'acide succinique;
- entre 0 et 75 %, de préférence entre 17 et 62 % en poids total de la composition d'éthanol;
- QSP eau ;

Par composition liquide, on entend désigner selon la présente invention, une composition ayant une viscosité comprise 0,002 et 2,5 Pa.S, de préférence entre 0,004 et 0,1 Pa.S.

La production de l'extrait de thé vert utilisé comme matière première est bien connue par l'homme de l'art. Par exemple, les feuilles de thé vert sont généralement extraites à l'eau chaude ou froide pour former une solution contenant des catéchines de thé et de la caféine. Cette solution de thé vert peut être davantage concentrée pour former soit une solution d'extrait concentré, soit une poudre sèche. La solution d'extrait ou la poudre peut contenir des stabilisants tels que des acides approuvés pour les aliments, par ex. l'acide citrique, l'acide ascorbique, l'acide isoascorbique et similaires. Les poudres d'extrait de thé sont également disponibles dans le commerce, par ex. de Guizhou Highyin Biological Product Co., Guiyang, P.R Chine, ou Zhejang Zhongke Plant Technical Co. Ltd., Hangzhou, Zhejang, P.R Chine.

Par catéchines, il est entendu selon la présente invention en particulier la (-)-épicatéchine (EC), la (-)-épigallocatéchine (EGC), le (-)-épigallocatéchine-3-gallate (EGCG) et le (-)-épicatéchine-3-gallate (ECg).

Il est entendu selon la présente invention que l'EGCG est le gallate d'épigallocatéchine qui est l'ester d'épigallocatéchine et d'acide gallique. En particulier l'EGCG est considéré comme l'ingrédient actif de la composition liquide d'enrichissement selon la présente invention et sera donc choisi par l'Homme du métier dans une quantité efficace pour l'effet souhaité, compris de préférence entre 9 et 20 % en poids total de la composition. Cela peut correspondre par exemple pour une capsule de café de 4,5g à 25g additionnée de 0,05 mL à 0,60 mL de composition liquide selon la présente invention à la quantité d'EGCG retrouvée dans de 1g à 2g de thé matcha.

Par acide dicarboxylique saturé ou insaturé ayant un maximum de 6 carbones est entendu au sens de l'invention des composés organiques possédant deux fonctions carboxyle ayant pour formule moléculaire HOOC-R-COOH, où R peut être un groupe alkyle, alcényle, alcynyle ou aryle. Ces composés sont facilement solubles dans l'eau, et respectent les normes imposées en matière d'utilisation en agroalimentaire. En effet, ceux-ci sont autorisés en tant qu'additif alimentaire, et autorisé pour leur utilisation cosmétique. Ledit acide dicarboxylique saturé ou insaturé possède une chaîne carbonée courte composée de maximum 6 carbones.

Par acide dicarboxylique saturé est entendu au sens de l'invention, un acide dicarboxylique linéaire saturé ayant pour formule générale HO₂C(CH₂)ₙCO₂H.

Par saturé est entendu que ledit acide dicarboxylique a un nombre total d'atomes égal à celui que l'on peut déduire de la valence maximale de chacun de ses atomes constitutifs pris individuellement (c'est-à-dire qu'il n'y a ni liaison double, ni liaison triple, ni cycle).

Ledit acide dicarboxylique saturé est l'acide succinique.

Par acide dicarboxylique insaturé est entendu un acide dicarboxylique ayant un nombre total d'atomes inférieur à celui que l'on peut déduire de la valence maximale de chacun de ses atomes constitutifs prise individuellement. Il peut ainsi comprendre notamment des liaisons doubles ou triples.

Des exemples d'acide dicarboxylique insaturé sont l'acide maléique, l'acide fumarique, l'acide cis-glutaconique, ou l'acide trans-glutaconique,

Ledit acide dicarboxylique saturé ou insaturé ayant un maximum de 6 carbones est l'acide succinique.

Par « ayant un maximum de 6 carbones » est entendu que l'acide dicarboxylique a un nombre de carbones compris entre 2 et 6.

Par acide succinique, on entend désigner selon la présente invention un diacide carboxylique aliphatique, dénommé également acide butane-1,4-dioïque. L'acide succinique en tant qu'additif alimentaire est listé comme régulateur d'acidité au Codex alimentarius, et comme exhausteur de goût d'origine naturelle. Il est listé sous le code E363 et est autorisé entre 3 et 6 grammes par kilo dans l'Union Européenne dans les produits laitiers fermentés, les soupes, les poudres pour préparations ménagères de boissons et les desserts. En dehors de l'alimentation transformée, on le trouve aussi dans les cosmétiques, notamment comme agent inhibiteur d'odeur ou de goût, et les produits pharmaceutiques.

L'acide succinique, permet de diminuer la densité et la viscosité de la composition liquide composée d'extrait de thé vert selon l'invention, qui en son absence est particulièrement dense et visqueuse, étant très fortement chargé dudit extrait de thé vert. Or, comme tout liquide très dense, sa pénétration dans la masse de café moulu déjà distribué dans la capsule est trop lente pour être compatible avec la cadence de fermeture des capsules. L'absence d'acide succinique résulterait en de fortes pertes dû à la mauvaise fermeture desdites capsules de café moulu.

De manière préférée, la composition liquide selon l'invention est caractérisée en ce que l'éthanol est de l'éthanol absolu, ou à 95% ou à 96%.

La composition liquide selon la présente invention comprend en outre au moins un excipient. L'Homme du métier choisira un ou des excipients acceptables en fonction du produit à enrichir au final et son utilisation. Ainsi pour un produit à enrichir à vocation alimentaire, il choisira des excipients acceptables selon les autorités de contrôles (FDA aux Etats-Unis, EFSA en Europe).

De manière préférée, la composition liquide selon l'invention est caractérisée en ce que l'extrait de thé vert comprend entre 10 et 40%, de préférence entre 15 et 35%, en poids total de la composition de catéchines.

Selon un mode de réalisation, la composition liquide d'enrichissement de café moulu comprend entre 34% et 44% en poids total de la composition de catéchines.

Selon un mode de réalisation, la composition liquide d'enrichissement de café moulu comprend entre 27% et 35% en poids total de la composition de catéchines.

Selon un mode de réalisation, la composition liquide d'enrichissement de café moulu comprend entre 37% et 48% en poids total de la composition de catéchines.

De manière préférée, la composition liquide selon l'invention est caractérisée en ce que l'extrait de thé vert comprend entre 5 et 25%, de préférence entre 9 et 20 % en poids total de la composition d'EGCG.

Selon un mode de réalisation, la composition liquide d'enrichissement de café moulu comprend entre 17% et 31% en poids total de la composition d'EGCG.

Selon un mode de réalisation, la composition liquide d'enrichissement de café moulu comprend entre 14% et 26% en poids total de la composition d'EGCG.

Selon un mode de réalisation, la composition liquide d'enrichissement de café moulu comprend entre 19% et 35% en poids total de la composition d'EGCG.

De manière préférée, la composition liquide selon l'invention est caractérisée en ce qu'elle est une solution ou une suspension hydro-alcoolique

Selon un deuxième objet, la présente invention concerne l'utilisation de la composition selon la présente invention pour l'enrichissement en extrait de thé vert, de manière préférée l'enrichissement en catéchines, de manière particulièrement préférée l'enrichissement en EGCG, de produits alimentaires à destination de l'être humain ou animal, de préférence du café moulu, de compléments alimentaires, de produits cosmétiques, et/ou de produits dermatologiques.

L'extrait de thé vert, de manière préférée les catéchines qu'il contient, de manière particulièrement préférée l'EGCG, présenté sous la forme d'une composition d'enrichissement liquide comme décrit ci-dessus possède une forte activité antioxydante et peut donc être utilisé comme antioxydant et/ou antivieillissement, pour divers produits alimentaires, aliments pour animaux, compléments alimentaires, produits cosmétiques, ou dermatologiques.

De plus, l'extrait de thé vert, de manière préférée les catéchines qu'il contient, de manière particulièrement préférée l'EGCG, a un effet antimutagène, un effet antibactérien et également un effet bénéfique sur le taux de cholestérol dans le sang. Ainsi, la composition liquide d'enrichissement selon la présente invention est utile dans les préparations de prévention ou de soin de la santé, en pharmaceutique ou parapharmaceutique, de préférence en complément alimentaire ou produit alimentaire enrichi, en particulier en prévention des pathologies cardiovasculaires, comme anti-cholestérol, prévention du cancer, prévention des infections de la peau, diminution du poids, augmentation du développement musculaire.

L'extrait de thé vert, de manière préférée les catéchines qu'il contient, de manière particulièrement préférée l'EGCG, a également un effet anxiolytique. Ainsi, la composition liquide d'enrichissement selon la présente invention est utile dans les préparations de prévention ou de soin de la santé, en pharmaceutique ou parapharmaceutique, de préférence en complément alimentaire ou produit alimentaire enrichi, en particulier pour la prévention ou diminution de l'anxiété, anti-stress, détente, prévention ou amélioration des troubles du sommeil, endormissement.

En outre l'extrait de thé vert, de manière préférée les catéchines qu'il contient, de manière particulièrement préférée l'EGCG, a des effets sur les fonctions endothéliales. Ainsi, la composition liquide d'enrichissement selon la présente invention est utile dans les préparations de prévention ou de soin de la santé, en pharmaceutique ou parapharmaceutique, de préférence en complément alimentaire ou produit alimentaire enrichi, en particulier pour le maintien des fonctions cognitives et de la mémoire, l'augmentation de la concentration et des facultés mentales, augmentation de la lucidité, de l'énergie, de la vivacité.

L'Homme du métier ajustera la quantité de composition liquide à ajouter au produit alimentaire, cosmétique ou dermatologique ou complément alimentaire selon ses connaissances générales de manière à obtenir le bénéfice souhaité. Ainsi selon un mode de réalisation, il peut être ajouté entre 0,1 et 40 **ml** de ladite composition liquide par kg de produit alimentaire, cosmétique ou dermatologique ou complément alimentaire pour un effet anti-oxydant. L'Homme du métier saura adapter la quantité de composition liquide à ajouter au produit afin d'obtenir une concentration efficace en catéchines et en particulier en EGCG, selon l'utilisation souhaitée et le bénéfice à obtenir, et qui ne dépasse pas les recommandations journalières des autorités de contrôle (en particulier FDA et EFSA).

Selon un mode de réalisation de l'invention, le produit alimentaire est une boisson. Selon un mode de réalisation de l'invention, le produit alimentaire est une poudre. Le produit alimentaire peut être en particulier une poudre (chocolat en poudre, lait en poudre, chicorée, boisson-repas...) prête à l'emploi pour la reconstitution d'une boisson après ajout d'un liquide (eau, lait) et dissolution.

De manière préférée, l'utilisation de la composition selon la présente invention est pour l'enrichissement en EGCG de café moulu compris dans une capsule de café à usage unique, de type compatible Nespresso^{®} ou de sachet-dosettes. Ces capsules contiennent traditionnellement entre 5 et 6g de café moulu pour la fabrication d'un expresso, et jusqu'à 25g pour la fabrication d'un café allongé de type lungo ou double expresso.

A titre d'exemple il peut être ajouté 0,16ml de la composition liquide selon l'invention, dosé entre 9 et 20% d'EGCG, dans 5 à 6g de café moulu dans une capsule de type expresso, et 0,48ml de la composition liquide selon l'invention dosé entre 9 et 20% d'EGCG, dans 15 à 20g de café moulu dans une capsule de type café allongé lungo.

Selon un autre exemple, il peut être ajouté 0,26 ml de la composition liquide selon l'invention, dosé entre 9 et 20% d'EGCG, dans 4,5 à 6 g de café moulu dans une capsule de type expresso, et 0,60ml de la composition liquide selon l'invention dosé entre 9 et 20% d'EGCG, dans 20 à 25g de café moulu dans une capsule prévue à cet effet.

Ces capsules de café moulu à usage unique sont utilisées pour réaliser une tasse de café grâce à l'utilisation combinée d'une machine à café, de préférence à haute-pression, et d'une capsule.

Les capsules sont par exemple faites d'une feuille d'aluminium et recouverte d'un film alimentaire interne qui permet d'éviter tout contact entre l'aluminium et le café. Dans une altemative les capsules sont faites en plastique ou en matériau composite biodégradable et compostable. Lorsque la capsule est insérée dans la machine, le haut est perforé. Puis, une fois la machine activée, l'eau est pompée pour être envoyée chaude et sous haute pression dans la capsule.

De manière préférée, selon la présente invention, chaque capsule comprenant entre 4,5 à 25 grammes de café moulu est enrichie par 0,05 ml à 0,60 millilitres de ladite composition selon l'invention.

Selon un mode de réalisation préféré, chaque capsule comprenant 4,5 à 25 grammes de café moulu est enrichie par 0,25 ml à 0,60 millilitres de ladite composition, de préférence 0,26ml.

Selon un autre mode de réalisation préféré, chaque capsule comprenant 5 à 6 grammes de café moulu est enrichie par 0,05 ml à 0,25 millilitres de ladite composition, de préférence entre 0,14 et 0,18 ml, de manière particulièrement préférée 0,16ml.

Selon un autre mode de réalisation préféré, l'utilisation de la composition selon la présente invention est caractérisée en ce que chaque capsule comprenant 4,5 à 25 grammes de café moulu est enrichie par 0,15 ml à 0,60 millilitres de ladite composition, de préférence 0,26 ml.

Selon un troisième objet, la présente invention concerne un procédé d'enrichissement de café moulu dans une capsule de café à usage unique comprenant notamment une étape d'ajout de la composition selon la présente invention audit café moulu avant fermeture de la capsule.

L'ajout de la composition liquide selon la présente invention est de préférence réalisé au moyen d'un système de distribution de liquide. Ce système est capable de délivrer des volumes précis de liquide grâce à une pompe ad-hoc générant un débit constant du liquide, dont la température nécessite d'être régulée pour optimiser sa liquidité.

Selon un quatrième objet, la présente invention concerne une capsule de café à usage unique enrichie en extrait de thé vert, de préférence en catéchines, de manière encore préférée en EGCG obtenue par le procédé selon la présente invention.

Selon un cinquième objet, la présente invention concerne un procédé de fabrication de la composition selon l'invention caractérisé en ce qu'il comprend les étapes suivantes :
a. Fabrication d'un système de solvant par mélange dudit éthanol, si présent dans la composition, et ladite eau
b. Ajout au système de solvant ainsi obtenu à l'étape a) dudit extrait de thé vert et mélange
c. Ajout au mélange obtenu à l'étape b) l'acide succinique, et mélange pour obtention d'un composition liquide.

On décrira maintenant, à titre d'exemple non limitatif, un mode de réalisation de l'invention

### EXEMPLES

### Exemple 1) Exemples de formulation de capsules de café à usage unique enrichie en extrait de thé vert contenant des catéchines, dont de l'EGCG

A. Composition liquide d'enrichissement de café moulu pour un effet amincissant

**[Table 1]**

| | quantité (mg) | % en poids |
|---|---|---|
| extrait de thé vert | 91 | 44,3 |
| dont catéchine | 68,25 | 33,2 |
| dont EGCG | 40 | 19,5 |
| acide succinique (E363) | 5 | 2,4 |
| Eau | 74,3 | 36,2 |
| ethanol 95 ou 95% | 35 | 17,1 |
| Volume total | 0,16ml | |
| total | 205,3 | 100 |

B. Composition liquide d'enrichissement de café moulu pour un effet favorisant la digestion

**[Table 2]**

| | quantité (mg) | % en poids |
|---|---|---|
| extrait de thé vert | 45,5 | 20,8 |
| dont catéchine | 34,1 | 15,6 |
| dont EGCG | 20 | 9,1 |
| acide succinique (E363) | 2,5 | 1,1 |
| Eau | 37,1 | 16,9 |
| ethanol 95 ou 95% | 134 | 61,2 |
| Volume total | 0,16ml | |
| Poids total | 219,1 | 100 |

C. Composition liquide d'enrichissement de café moulu pour un effet anti-cholestérol

**[Table 3]**

| | quantité (mg) | % en poids |
|---|---|---|
| extrait de thé vert | 68,25 | 32,2 |
| dont catéchine | 51,19 | 24,1 |
| dont EGCG | 30 | 14,1 |
| acide succinique (E363) | 3,7 | 1,7 |
| Eau | 55,7 | 26,3 |
| ethanol 95 ou 95% | 84,4 | 39,8 |
| Volume total | 0,16ml | |
| total | 212,05 | 100 |

D. Composition liquide d'enrichissement de café moulu pour un effet anti-âge

**[Table 4]**

| | quantité (mg) | % en poids |
|---|---|---|
| extrait de thé vert | 68,25 | 32,2 |
| dont catéchine | 51,19 | 24,1 |
| dont EGCG | 30 | 14,1 |
| acide succinique (E363) | 3,7 | 1,7 |
| Eau | 55,7 | 26,3 |
| ethanol 95 ou 95% | 84,4 | 39,8 |
| Volume total | 0,16ml | |
| total | 212,05 | 100 |

### 2) Enrichissement de la capsule de café

Chaque volume de solution de 0,16ml (Table 1, 2, 3, 4) est additionné dans une capsule de type Nespresso^{®} pour la fabrication d'un expresso, sur le café fraichement moulu. Il est constaté une évaporation du liquide en quelques secondes, et la formation d'un agrégat de café sec à l'endroit où la composition liquide a été additionnée. La capsule est scellée, prête à l'emploi.

### 3) Obtention d'une tasse de café enrichi avec bénéfice pour le consommateur

La capsule ainsi obtenue est insérée dans une machine de type Nespresso^{®}, ajout d'eau chaude sous pression, pour réaliser une tasse de café enrichi en extrait de thé vert, catéchines et EGCG, avec obtention du bénéfice indiqué (amincissant, facilitant la digestion, anti-cholestérol, anti-âge) pour le consommateur.

### Exemple 2) Exemples de formulation de capsules de café à usage unique enrichie en extrait de thé vert contenant des catéchines, dont de l'EGCG

A. Composition liquide d'enrichissement de café moulu pour un effet amincissant
Les pourcentages en poids des tableaux 5 à 8 sont exprimés pour la totalité de la composition obtenue dans la capsule (café + composition selon l'invention).

**[Table 5]**

| | quantité (mg) | % en poids (arrondi à 0,01) |
|---|---|---|
| extrait de thé vert | 139,1 | 2,64 |
| dont catéchine | 107 | 2,02 |
| dont EGCG | 58 | 1,10 |
| acide succinique (E363) | 1,3 | 0,03 |
| Eau | 90 | 1,70 |
| ethanol 95 ou 95% | 57,0 | 1,10 |
| Volume total | 0,26ml | |
| Capsule de 5g | 5 000 | 94,60 |
| total | 5 287 | 100 |

B. Composition liquide d'enrichissement de café moulu pour un effet favorisant la digestion

**[Table 6]**

| | quantité (mg) | % en poids (arrondi à 0,01) |
|---|---|---|
| extrait de thé vert | 106 | 2,01 |
| dont catéchine | 82 | 1,56 |
| dont EGCG | 44 | 0,83 |
| acide succinique (E363) | 1 | 0,02 |
| Eau | 80 | 1,52 |
| ethanol 95 ou 95% | 83 | 1,57 |
| Volume total | 0,26ml | |
| Capsule de 5g | 5 000 | 94,90 |
| total | 5270 | 100 |

C. Composition liquide d'enrichissement de café moulu pour un effet anti-cholestérol

**[Table 7]**

| | quantité (mg) | % en poids (arrondi à 0,01) |
|---|---|---|
| extrait de thé vert | 139,1 | 2,63 |
| dont catéchine | 107 | 2,02 |
| dont EGCG | 58 | 1,10 |
| acide succinique (E363) | 1,3 | 0,02 |
| Eau | 90 | 1,70 |
| ethanol 95 ou 95% | 57,0 | 1,10 |
| Volume total | 0,26ml | |
| Capsule de 5g | 5000 | 94,60 |
| total | 5 287 | 100 |

D. Composition liquide d'enrichissement de café moulu pour un effet anti-âge

**[Table 8]**

| | quantité (mg) | % en poids (arrondi à 0,01) |
|---|---|---|
| extrait de thé vert | 159 | 3,00 |
| dont catéchine | 122 | 2,30 |
| dont EGCG | 66 | 1,25 |
| acide succinique (E363) | 1,5 | 0,03 |
| Eau | 100 | 1,90 |
| ethanol 95 ou 95% | 38 | 0,72 |
| Volume total | 0,26ml | |
| Capsule de 5g | 5 000 | 94,36 |
| total | 5 299 | 100 |

### 2) Enrichissement de la capsule de café

Chaque volume de solution de 0,26ml (Table 5, 6, 7 et 8) est additionné dans une capsule de type Nespresso^{®} de 5g, pour la fabrication d'un café de type expresso, sur le café fraichement moulu. Il est constaté une évaporation du liquide en quelques secondes, et la formation d'un agrégat de café sec à l'endroit où la composition liquide a été additionnée. La capsule est scellée, prête à l'emploi.

### 3) Obtention d'une tasse de café enrichi avec bénéfice pour le consommateur

La capsule ainsi obtenue est insérée dans une machine de type Nespresso^{®}, ajout d'eau chaude sous pression, pour réaliser une tasse de café enrichi en extrait de thé vert, catéchines et EGCG, avec obtention du bénéfice indiqué (amincissant, facilitant la digestion, anti-cholestérol, anti-âge) pour le consommateur.

### Exemple 3) Exemples de formulation de capsules de café à usage unique enrichie en extrait de thé vert contenant des catéchines, dont de l'EGCG

A. Composition liquide d'enrichissement de café moulu pour un effet amincissant Les pourcentages en poids des tableaux 9 à 12 sont exprimés pour la totalité de la composition obtenue dans la capsule (café + composition selon l'invention).

**[Table 9]**

| | quantité (mg) | % en poids (arrondi à 0,01) |
|---|---|---|
| extrait de thé vert | 139,1 | 0,55 |
| dont catéchine | 107 | 0,42 |
| dont EGCG | 58 | 0,3 |
| acide succinique (E363) | 1,3 | 0,01 |
| Eau | 90 | 0,36 |
| ethanol 95 ou 95% | 57,0 | 0,23 |
| Volume total | 0,26ml | |
| Capsule de 25g | 25 000 | 98,86 |
| total | 25 287 | 100 |

B. Composition liquide d'enrichissement de café moulu pour un effet favorisant la digestion (ne fait pas partie de l'invention)

**[Table 10]**

| | quantité (mg) | % en poids (arrondi à 0,0001) |
|---|---|---|
| extrait de thé vert | 106 | 0,4195 |
| dont catéchine | 82 | 0,3245 |
| dont EGCG | 44 | 0,1741 |
| acide succinique (E363) | 1 | 0,0004 |
| Eau | 80 | 0,3166 |
| ethanol 95 ou 95% | 83 | 0,3285 |
| Volume total | 0,26ml | |
| Capsule de 25g | 25 000 | 98,9315 |
| total | 25 270 | 100 |

C. Composition liquide d'enrichissement de café moulu pour un effet anti-cholestérol

**[Table 11]**

| | quantité (mg) | % en poids (arrondi à 0,01) |
|---|---|---|
| extrait de thé vert | 139,1 | 0,55 |
| dont catéchine | 107 | 0,42 |
| dont EGCG | 58 | 0,23 |
| acide succinique (E363) | 1,3 | 0,01 |
| Eau | 90 | 0,36 |
| ethanol 95 ou 95% | 57,0 | 0,23 |
| Volume total | 0,26ml | |
| Capsule de 25g | 25 000 | 98,86 |
| total | 25 287 | 100 |

D. Composition liquide d'enrichissement de café moulu pour un effet anti-âge

**[Table 12]**

| | quantité (mg) | % en poids (arrondi à 0,01) |
|---|---|---|
| extrait de thé vert | 159 | 0,63 |
| dont catéchine | 122 | 0,48 |
| dont EGCG | 66 | 0,26 |
| acide succinique (E363) | 1,5 | 0,01 |
| Eau | 100 | 0,40 |
| ethanol 95 ou 95% | 38 | 0,15 |
| Volume total | 0,26ml | |
| Caspule de 25g | 25 000 | 98,82 |
| total | 25 299 | 100 |

### 2) Enrichissement de la capsule de café

Chaque volume de solution de 0,26ml (Table 9, 10, 11 et 12) est additionné dans une capsule de 25g, pour la fabrication d'un café allongé, sur le café fraichement moulu. Il est constaté une évaporation du liquide en quelques secondes, et la formation d'un agrégat de café sec à l'endroit où la composition liquide a été additionnée. La capsule est scellée, prête à l'emploi.

### 3) Obtention d'une tasse de café enrichi avec bénéfice pour le consommateur

La capsule ainsi obtenue est insérée dans une machine de type Nespresso^{®}, ajout d'eau chaude sous pression, pour réaliser une tasse de café enrichi en extrait de thé vert, catéchines et EGCG, avec obtention du bénéfice indiqué (amincissant, facilitant la digestion, anti-cholestérol, anti-âge) pour le consommateur.

## Revendications

1. Composition liquide comprenant :
- entre 15 et 60 %, de préférence entre 20 et 45%, en poids total de la composition d'extrait de thé vert, ledit extrait comprenant entre 10 et 50%, de préférence entre 10 et 40%, de manière plus préférée, entre 15 et 35%, en poids total de la composition de catéchines, comprenant entre 5 et 40%, de préférence entre 5 et 25%, de manière plus préférée entre 9 et 20 % en poids total de la composition d'EGCG;
- entre 0,5 et 5,5%, de préférence entre 1 et 3 % en poids total de la composition d'acide succinique;
- entre 0 et 75 %, de préférence entre 17 et 62 % en poids total de la composition d'éthanol;
- QSP eau ;

2. Composition liquide selon la revendication 1 **caractérisée en ce que** l'extrait de thé vert comprend entre 10 et 40%, de préférence entre 15 et 35%, en poids total de la composition de catéchines.

3. Composition liquide selon la revendication 1 ou 2 **caractérisée en ce que** l'extrait de thé vert comprend entre 5 et 25%, de préférence entre 9 et 20 % en poids total de la composition d'EGCG.

4. Composition liquide selon l'une quelconque des revendications 1 à 3 **caractérisée en ce qu'**elle est une solution ou une suspension hydro-alcoolique

5. Composition liquide selon l'une quelconque des revendications 1 à 4 **caractérisée en ce que** l'éthanol est de l'éthanol absolu, ou à 95% ou à 96%.

6. Utilisation de la composition selon l'une quelconque des revendications 1 à 5 pour l'enrichissement en extrait de thé vert, de manière préférée l'enrichissement en catéchine, de manière particulièrement préférée **l'enrichissement** en EGCG de produits alimentaires à destination de **l'être** humain ou animal, de préférence du café moulu, de compléments alimentaires de l'être humain ou animal, de produits cosmétiques, et/ou de produits dermatologiques.

7. Utilisation selon la revendication 6 de ladite composition pour l'enrichissement en EGCG de café moulu compris dans une capsule de café à usage unique.

8. Utilisation selon la revendication 7 **caractérisée en ce que** chaque capsule comprenant 4,5 à 25 grammes de café moulu est enrichie par 0,05 **ml** à 0,60 millilitres de ladite composition.

9. Utilisation selon la revendication 8 **caractérisée en ce que** chaque capsule comprenant 4,5 à 25 grammes de café moulu est enrichie par 0,15 **ml** à 0,60 millilitres de ladite composition, de préférence 0,26ml.

10. Utilisation selon la revendication 9 **caractérisée en ce que** chaque capsule comprenant 5 à 6 grammes de café moulu est enrichie par 0,05 **ml** à 0,25 millilitres de ladite composition, de préférence 0,16ml.

11. Utilisation selon la revendication 9 **caractérisée en ce que** chaque capsule comprenant 4,5 à 25 grammes de café moulu est enrichie par 0,25 **ml** à 0,60 millilitres de ladite composition, de préférence 0,26ml.

12. Procédé d'enrichissement de café moulu dans une capsule de café à usage unique comprenant notamment une étape d'ajout de la composition selon l'une quelconque des revendications 1 à 5 audit café moulu avant fermeture de la capsule.

13. Capsule de café à usage unique enrichie en extrait de thé vert, de préférence en catéchines, de manière encore préférée en EGCG, grâce à la composition selon l'une quelconque des revendications 1 à 5.

14. Procédé de fabrication de la composition selon l'une quelconque des revendications 1 à 5 **caractérisé en ce qu'**il comprend les étapes suivantes :
a. fabrication d'un système de solvant par mélange dudit éthanol, si présent dans la composition, et ladite eau
b. Ajout au système de solvant ainsi obtenu à l'étape a) dudit extrait de thé vert et mélange
c. Ajout au mélange obtenu à l'étape b) dudit acide succinique, et mélange pour obtention d'un composition liquide.

## Patentansprüche

1. Flüssige Zusammensetzung, umfassend:
- zwischen 15 und 60 Gew.-%, vorzugsweise zwischen 20 und 45 Gew.-%, bezogen auf das Gesamtgewicht der Zusammensetzung an Grüntee-Extrakt, der Extrakt umfassend zwischen 10 und 50 Gew.-%, vorzugsweise zwischen 10 und 40 Gew.-%, mehr bevorzugt zwischen 15 und 35 Gew.-%, bezogen auf das Gesamtgewicht der Zusammensetzung an Catechinen, umfassend zwischen 5 und 40 Gew.-%, vorzugsweise zwischen 5 und 25 Gew.-%, mehr bevorzugt zwischen 9 und 20 Gew.-%, bezogen auf das Gesamtgewicht der Zusammensetzung an EGCG;
- zwischen 0,5 und 5,5 %, vorzugsweise zwischen 1 und 3 Gew.-%, bezogen auf das Gesamtgewicht der Zusammensetzung an Bernsteinsäure;
- zwischen 0 und 75 %, vorzugsweise zwischen 17 und 62 Gew.-%, bezogen auf das Gesamtgewicht der Zusammensetzung an Ethanol;
- QSP Wasser;

2. Flüssige Zusammensetzung nach Anspruch 1,
**dadurch gekennzeichnet, dass** der Grüntee-Extrakt zwischen 10 und 40 %, vorzugsweise zwischen 15 und 35 %, bezogen auf das Gesamtgewicht der Zusammensetzung an Catechinen umfasst.

3. Flüssige Zusammensetzung nach Anspruch 1 oder 2,
**dadurch gekennzeichnet, dass** der Grüntee-Extrakt zwischen 5 und 25 %, vorzugsweise zwischen 9 und 20 Gew.-% der Gesamtzusammensetzung an EGCG umfasst.

4. Flüssige Zusammensetzung nach einem der Ansprüche 1 bis 3,
**dadurch gekennzeichnet, dass** sie eine hydroalkoholische Lösung oder Suspension ist

5. Flüssige Zusammensetzung nach einem der Ansprüche 1 bis 4,
**dadurch gekennzeichnet, dass** das Ethanol absolutes Ethanol oder 95 %iges oder 96 %iges Ethanol ist.

6. Verwendung der Zusammensetzung nach einem der Ansprüche 1 bis 5 für die Anreicherung mit Grüntee-Extrakt, bevorzugt für die Anreicherung mit Catechin, noch mehr bevorzugt für die Anreicherung mit EGCG von Nahrungsmitteln für den Menschen oder das Tier, vorzugsweise gemahlenem Kaffee, von Nahrungsergänzungsmitteln für den Menschen oder das Tier, von kosmetischen Produkten und/oder von dermatologischen Produkten.

7. Verwendung nach Anspruch 6 der Zusammensetzung für die Anreicherung von gemahlenem Kaffee, der in einer Einwegkaffeekapsel enthalten ist, mit EGCG.

8. Verwendung nach Anspruch 7, **dadurch gekennzeichnet, dass** jede Kapsel, umfassend 4,5 bis 25 Gramm gemahlenen Kaffee, mit 0,05 ml bis 0,60 Milliliter der Zusammensetzung angereichert ist.

9. Verwendung nach Anspruch 8, **dadurch gekennzeichnet, dass** jede Kapsel, umfassend 4,5 bis 25 Gramm gemahlenen Kaffee, mit 0,15 ml bis 0,60 Milliliter der Zusammensetzung angereichert ist, vorzugsweise 0,26 ml.

10. Verwendung nach Anspruch 9, **dadurch gekennzeichnet, dass** jede Kapsel, umfassend 5 bis 6 Gramm gemahlenen Kaffee, mit 0,05 ml bis 0,25 Milliliter der Zusammensetzung angereichert ist, vorzugsweise 0,16 ml.

11. Verwendung nach Anspruch 9, **dadurch gekennzeichnet, dass** jede Kapsel, umfassend 4,5 bis 25 Gramm gemahlenen Kaffee, mit 0,25 ml bis 0,60 Milliliter der Zusammensetzung angereichert ist, vorzugsweise 0,26 ml.

12. Verfahren für die Anreicherung von gemahlenem Kaffee in einer Einwegkaffeekapsel, das insbesondere einen Schritt eines Zugebens der Zusammensetzung nach einem der Ansprüche 1 bis 5 zu gemahlenen Kaffee vor einem Verschließen der Kapsel umfasst.

13. Einwegkaffeekapsel, die dank der Zusammensetzung nach einem der Ansprüche 1 bis 5 mit Grüntee-Extrakt, vorzugsweise mit Catechinen, noch mehr bevorzugt mit EGCG angereichert ist.

14. Verfahren zum Herstellen der Zusammensetzung nach einem der Ansprüche 1 bis 5, **dadurch gekennzeichnet, dass** es die folgenden Schritte umfasst:
a. Herstellen eines Lösungsmittelsystems durch Mischen des Ethanols, falls in der Zusammensetzung vorhanden, und des Wassers
b. Zugeben zu dem so in Schritt a) erhaltenen Lösungsmittelsystem des Grüntee-Extrakts und Mischen
c. Zugeben der Bernsteinsäure zu der in Schritt b) erhaltenen Mischung und Mischen zum Erhalten einer flüssigen Zusammensetzung.

## Claims

1. **A liquid** composition comprising:
∘ between 15 and 60%, preferably between 20 and 45%, based on the total weight of the composition, of green tea extract, said extract comprising between 10 and 50%, preferably between 10 and 40%, more preferably between 15 and 35%, based on the total weight of the composition, of catechins, comprising between 5 and 40%, preferably between 5 and 25%, more preferably between 9 and 20%, based on the total weight of the composition, of EGCG;
∘ between 0.5 and 5.5%, preferably between 1 and 3%, based on the total weight of the composition, of succinic acid;
∘ between 0 and 75%, preferably between 17 and 62%, based on the total weight of the composition, of ethanol;
∘ QSP water.

2. The liquid composition according to claim 1, **characterized in that** the green tea extract comprises between 10 and 40%, preferably between 15 and 35%, based on the total weight of the composition, of catechins.

3. The liquid composition according to claim 1 or 2, **characterized in that** the green tea extract comprises between 5 and 25%, preferably between 9 and 20%, based on the total weight of the composition, of EGCG.

4. The liquid composition according to any one of claims 1 to 3, **characterized in that** it is a hydro-alcoholic solution or suspension.

5. The liquid composition according to any one of claims 1 to 4, **characterized in that** the ethanol is absolute ethanol, or 95% or 96% ethanol.

6. Use of the composition according to any one of claims 1 to 5 for the enrichment with green tea extract, preferably the enrichment with catechin, particularly preferably the enrichment with EGCG, of food products intended for human beings or animals, preferably ground coffee, of food supplements for human beings or animals, of cosmetic products, and/or of dermatological products.

7. The use according to claim 6 of said composition for the enrichment with EGCG of ground coffee comprised in a single-use coffee capsule.

8. The use according to claim 7, **characterized in that** each capsule comprising 4.5 to 25 grams of ground coffee is enriched with 0.05 ml to 0.60 milliliters of said composition.

9. The use according to claim 8, **characterized in that** each capsule comprising 4.5 to 25 grams of ground coffee is enriched with 0.15 ml to 0.60 milliliters of said composition, preferably 0.26 ml.

10. The use according to claim 9, **characterized in that** each capsule comprising 5 to 6 grams of ground coffee is enriched with 0.05 ml to 0.25 milliliters of said composition, preferably 0.16 ml.

11. The use according to claim 9, **characterized in that** each capsule comprising 4.5 to 25 grams of ground coffee is enriched with 0.25 ml to 0.60 milliliters of said composition, preferably 0.26 ml.

12. A method for enriching ground coffee in a single-use coffee capsule comprising in particular a step of adding the composition according to any one of claims 1 to 5 to said ground coffee before sealing the capsule.

13. A single-use coffee capsule enriched with green tea extract, preferably with catechins, more preferably with EGCG, through the composition according to any one of claims 1 to 5.

14. A method for producing the composition according to any one of claims 1 to 5, **characterized in that** it comprises the following steps:
a. producing a solvent system by mixing said ethanol, if present in the composition, and said water
b. adding to the solvent system thus obtained in step a) said green tea extract and mixing
c. adding to the mixture obtained in step b) said succinic acid, and mixing to obtain a liquid composition.
